# EUROPEAN PATENT APPLICATION

(11) **EP 1 548 099 A1**
(43) Date of publication of application: **29.06.2005**
(21) Application number: 03771452.4
(22) Date of filing: 31.07.2003
(51) Int. Cl.: C12M 3/00

(54) **AUTOMATIC CULTURE APPARATUS FOR CELL OR TISSE WITH BIOLOGICAL ORIGIN**

(30) Priority: 31.07.2002 JP 2002223892; 10.03.2003 JP 2003064155
(71) Applicant: Japan Science and Technology Agency, Kawaguchi-shi, Saitama 332-0012 (JP)
(72) Inventor: TAKAGI, Mutsumi, Ibaraki-shi, Osaka 567-0046 (JP); YOSHIDA, Toshiomi, Suita-shi, Osaka 565-0824 (JP); WAKITANI, Shigeyuki, Matsumoto-shi, Nagano 390-0304 (JP)
(74) Representative: Calamita, Roberto
(86) International application number: PCT/JP2003/009742
(87) International publication number: WO 2004/011593

(57) **Abstract**

An automatic culture apparatus for culturing cells or a tissue with a biological origin in a culture container which is placed in a box-type culture apparatus having a closed and aseptic inner spade, **characterized in that** a plural number of divided spaces, a gas incubator provided with a practicable window, a unit for supplying a liquid culture medium and a unit for discharging the same, a unit for monitoring the culture conditions, and a transferring unit for continuously or intermittently transferring the culture container toward these units are provided in the box of the culture apparatus, and at least one of the above-described units is equipped with an instruction controlling unit by which the culture apparatus is controlled under an instruction in the form of an electrical signal depending on a data signal generated by the culture condition-monitoring unit so as to prevent cross-contamination.

## Description

### Technical Field

The invention of the present application relates to an automatic culture apparatus for culturing cells or a tissue. More specifically, the invention of the present application relates to a culture apparatus in which an operation of culturing cells or a tissue and control of culture environments are automated intending regenerative medicine and the like.

Further, the invention of the present application relates to an automatic culture apparatus equipped with a measuring apparatus which is noninvasive for cells or a tissue.

### Background Art

Recently, regenerative medicine in which cells and a tissue obtained by culturing organism cells and an organism tissue in vitro are used for repair of deficiency and defective and incomplete regions in a body or on the surface of a body shows enhanced actual possibility owing to accumulation and development of a lot of basic studies, and also provides a significant social expectation. Actually, studies to date report that a lot of tissues of skin, cartilage, bone, blood vessel, liver cell, pancreas and the like can be an object of regenerative medicine. Origins of cells or tissues for such regenerative medicine include differentiated tissues of skin, cartilage and the like or cells in these tissues, hematopoietic stem cells said to be present in medulla fluid and the like, somatic stem cells such as mesenchymal stem cells, liver stem cells present in liver, and the like, further, embryonic stem cells (ES cells) originated from a cell block in a fertilized egg and having an ability to differentiate into cells of almost all tissues in a body, and the like.

The number of cells with any origin obtained from an organism is limited, therefore, when these are used for regenerative medicine, it is generally necessary to culture, proliferate and differentiate them. Consequently, in the case, for example, of skin derived form epidermic cell, cartilage derived from cartilage cell, and the like, it is believed necessary to proliferate them while maintaining their differentiated condition, and in the case of use of somatic stem cells or embryonic stem cells, it is believed necessary to proliferate stem cells, then, to differentiate them into cells depending to a remedy region. Furthermore, there is also a method suggested in which stem cells are proliferated to increase the cell number, the cells are transplanted into an organism, and differentiation and tissue formation are performed in the organism.

On the other hand, derivation of cells or a tissue of an organism includes a case of use of cells or a tissue of a subject himself, and a case of use of cells or a tissue derived from a human other than a subject himself. The former case has a merit that a possibility of a rejection reaction in transplantation is low, while has a necessity that cells or a tissue as a material is prepared for each patient to undergo transplantation. In the latter case, there is a possibility of conducting regenerative medicine on a lot of patients using cells or tissues with the same individual origin, however, because of differences in medical care contents such as the shape and size of regeneration tissues necessary for patients, it is necessary to culture cells or tissues in difference lots for respective patients. Further, culturing cells or a tissue intending regenerative medicine has a feature that its culture scale is extremely small. For example, the culture scale is believed to be 100 ml or less when mesenchymal stem cells contained in 10 ml of medulla fluid are proliferated, then, differentiated into cartilage cells, and used for regenerative medicine of cartilage. Namely, culturing cells or a tissue intending regenerative medicine requires "small scale and multiple lot concurrent culture".

In culturing cells or a tissue, it is necessary to strictly make a caution and countermeasure for preventing pollution by viruses, bacteria, various chemical substances and the like. When regenerative medicine such as transplantation and the like is intended, viruses, bacteria, various chemical substances and the like are mixed, and when pollution occurs, there is a possibility of remarkable change in nature such as canceration of cells or a tissue during culture, and the like, and transplantation of cells or a tissue having changed nature into an organism gives a possibility of a novel disease such as induction of canceration and the like, and of a novel pathogenesis of viruses, bacteria and the like causing pollution, therefore, prevention of pollution should be strictly performed. That is, it is necessary that culture of cells or a tissue is conducted in a facility having a strict management organization and culture operation standards such as a highly sterilized operation and the like are made. For example, in National Institute of Advanced Industrial Science and Technology, an operator wears a dust free garment in a class 1000 clean room, and performs culture by a highly sterilized operation using a class 100 clean bench.

As indicated by Food and Drug Administration (FDA)(respect to be considered for management of quality and production of medical product for cell treatment using human somatic cell), cells and tissues of a patient itself (autocyto), among cells and tissues with biological origin, show different size and shape of a regeneration tissue required depending on the patient, and it should be avoided without fail to mix a pollution source carried intrinsically on the patient with cell and tissue culture originated from other patients.

Culturing of cells or a tissue intending regenerative medicine needs a long period of time. In general, the speed of proliferating culture cells derived from animals such as mammals and the like is slow proliferation speed in vitro, and need a time of 2 to 3 days for multiplication of cell number. On the other hand, the speed of proliferating miscellaneous germs such as bacteria and the like is slow, and its multiplication time is only about 20 minutes to 1 hour, consequently, when only one miscellaneous germ such as a bacterium or the like is mixed, proliferation of the miscellaneous germ such as a bacterium or the like is more significant than proliferation of cells or a tissue during culture, and the cells or a tissue during culture cannot be used at all.

For solving the problems as described above, many improvement measures have been planed and tried, however, the following problems still remain.
(1) A possibility of pollution is present though very slight, due to handwork;
(2) A possibility of an operator itself of becoming a pollution source of virus, mycoplasma, bacterium and the like;
(3) A special culture facility is necessary in which conditions, environments and the like are maintained like under strict management;
(4) An operating efficiency is low;
(5) Since high skill is necessary for operation, operators are limited, to increase personnel expenditure;
(6) In the case of autocyto, a plural number of cell species are operated simultaneously in the same facility, therefore, there is a possibility of mutual mixing;

For solving these problems, there have been developed an apparatus comprising a culture chamber capable of arbitrarily controlling environmental conditions, and a liquid culture medium storage container and a waste liquid culture medium storage container connected by piping to the chamber (JP 2001-238663, "Cell mate" manufactured by Automation Partnership (US), "Select T" manufactured by the same company, and the like. However, all of these apparatuses have a problem that there is no feature of "small scale and multiple lot concurrent culture" as a feature required for culturing cells or a tissue intending regenerative medicine, a problem that dedicated culture containers are necessary and a plural number of culture operations cannot be carried out simultaneously only with one culture apparatus, a problem of maintenance such as periodic washing and the like in culturing for a long period of time, a problem that culture conditions cannot be arbitrarily changed during culture, and the like.

Further, most animal cells has such adhesion dependency that they cannot survive without physical adhesion to a certain substrate. Therefore, in general, cells are adhered to the bottom of a culture container made of plastic and the like and cultured. When proliferated and differentiated cells are transplanted to an organism, and the like, and cells after culturing are used for utilization in regenerative medicine, it is necessary to peel cells from a culture container to give a floating condition except some cases using cells under condition of adhesion to the culture container. Conventionally, proteases such as trypsin, collagenase and the like are used for detaching and peeling of such cells. However, it is difficult to completely peel cells only by an enzymatic treatment, and even if cells are peeled from the surface of a culture container, aggregates of cells remain frequently. Then, in such a case, a pipetting operation is usually conducted in which an operation of spraying a liquid culture medium or washing liquid on the cell adhesion surface of a culture container and causing peeling or dissociation of a cell block is repeated. This pipetting operation is important in a culture operation, however, operators are limited since the operation needs skills such as control of spray speed, control of angle formed by spray direction and a cell adhesion surface in a culture container, and the like. As methods of injecting a liquid culture medium and the like aseptically into a culture container, there are, for example, the following methods.
(1) A tube set on a liquid culture medium container is squeezed by a tubing pump, to inject a liquid culture medium into a culture container;
(2) Instruments such as a pipette and the like are inserted into a liquid culture medium container, a liquid culture medium is collected, and this liquid culture medium is injected into a culture container;

However, such injection methods provide also a possibility in which in injecting a liquid culture medium into a culture container, a culture substance adheres to the tip of a tube, pipette and the like, and mixes into another culture container, and a possibility of mutual mixing via a gas for collecting a liquid culture medium into an instrument such as a pipette or the like. Additionally, when this culture substance is a different cell species, or when a bacterium or causative factor adheres to a culture substance, its influence may possibly be a problem. In culturing intending regenerative medicine, its influence is particularly large since multiple lot concurrent culture is useful.

Even in cell culture, it is important to control culture environments mimicking physiological environments under which tissues and cells are placed in an organism, for obtaining cells having high function and activity, however, this environmental factor in an organism includes physical forces such as tension, shear strength, hydrostatic pressure, pressing force and the like. For example, it is known that vascular endothelial cells deform by shear strength by vascular flow, and cytokine production amount varies. Conventionally, there are a lot of reports for apparatuses and methods of controlling culture environments regarding tension, shear strength and hydrostatic pressure, however, there is no example of an apparatus for controlling pressing force.

On the other hand, in such a culture process, analysis and measurement of culturing progress of a culture substance are also important. Particularly, analysis and measurement of (1) "the amount" of cells and tissues during culture and (2) "the quality" of cells and tissues during culture are important for continuation or termination of culture or variation of culture conditions, and the like. For example, by measuring the quantity change of cells and tissues with the lapse of time, suitable culture operations (judge of continuation of culture and completion of culture, and the like) can be conducted smoothly, and by analyzing and measuring quality information such as the metabolic activity, viability and differentiated condition of cells being cultured, and the like, conditions of cells can also be grasped correctly. However, as conventional such measurement means, observation by an optical microscope and the like from one direction (mainly, from upper side of culture container), of cells and tissues in a culture container and image analysis thereof are general, and by such measurement means, only information obtained from one direction (two-dimensional information), namely, information on the sum of areas of cell adhesion (cell adhesion surface) can be obtained. Further, the thickness of a cell adhesion layer is various, therefore, the quantity measurement of cells and tissues during culture is difficult only by image analysis of an optical microscope observation image.

Further, when cells and tissues are cultured for the purpose of regenerative medicine and the like, it is necessary to conduct multiple lot culture for many times, for measuring cells and the like by a conventional sampling examination (invasion examination), owing to "small scale and multiple lot concurrent culture" as described above. When cells are broken before examination and measurement such as by an invasion examination, production efficiency significantly lowers. Furthermore, because of an object of transplantation to a patient body, quality management stricter than for medicine production is required, therefore, it is important that direct contact of instruments and apparatuses for measurement with cells, tissues and liquid culture medium during culture and the like is avoided as completely as possible. Accordingly, for cell culture intending regenerative medicine, an automated culture apparatus is useful, further, measurement means characterized in noninvasion (namely, non-contact, non-broken) and automated are also expected to be useful.

As the noninvasive measurement method, there have been developed "Laser Focus displacement meter" manufactured by KEYENCE, "wavelength con-focal mode" by Nihon Binary Co.,Ltd, and the like. However, these noninvasive measurement apparatuses have no example of standing adhesion culture mode, and have no example in which analysis and measurement works are automated with a series of culturing operations.

The invention of the present application has, in view of the above-mentioned conditions, to enable culturing for a long period of time, an object of providing a novel culture apparatus which does not need special sterilization facilities as conventionally used, can effect culturing under an extremely clean environment, does not depend on limited operators having high skills, and can optionally control automatically pressing force and the like, environmental factors of culture environments, depending on culture progress, efficiently and with easy maintenance. Also, the invention of the present application has an object of providing a novel automatic culture apparatus also enabling prevention of mutual mixing of different cell species and tissue species.

Further, the invention of the present application has an object of providing a novel automatic culture apparatus equipped with a measurement apparatus which can noninvasively and three-dimensionally analyze and measure the amount and/or quality of cells or a tissue with biological origin.

### Disclosure of Invention

The present application has been made as an invention for solving the above-mentioned problems, and provides a culture apparatus in which a series of culture operations are automated in a closed and aseptic culture apparatus. Also, the present application provides a measurement apparatus in which operations for noninvasively and three-dimensionally analyzing and measuring the amount and/or quality of cells and a tissue are automated.

Namely, the invention of the present application provides, in a first aspect, an automatic culture apparatus for culturing cells or a tissue with a biological origin in a culture container which is placed in a box-type culture apparatus having a closed and aseptic inner space, characterized in that a plural number of divided spaces, a gas incubator provided with a practicable window, a unit for supplying a liquid culture medium and a unit for discharging the same, a unit for monitoring the culture conditions, and a transferring unit for continuously or intermittently transferring the culture container toward these units are provided in the box of the culture apparatus, and at least one of the above-described units is equipped with an instruction controlling unit by which the culture apparatus is controlled under an instruction in the form of an electrical signal depending on a data signal generated by the culture condition-monitoring unit so as to prevent cross-contamination.

The invention of the present application provides, in a second aspect, the automatic culture apparatus, wherein the apparatus has a setting unit for introducing a sterile gas into all portions or partial portions in the box of the culture apparatus, in a third aspect, the automatic culture apparatus, wherein the sterile gas is an ozone gas, and in a fourth aspect, the automatic culture apparatus, wherein the apparatus has an environmental condition setting unit for allowing all portions or partial portions in the box of the culture apparatus to have a positive pressure higher than that of the outer space.

The invention of the present application provides, in a fifth aspect, the automatic culture apparatus, wherein the box of the culture apparatus is divided into a plural number of spaces and the spaces are mutually closable, and in a sixth aspect, the automatic culture apparatus, wherein the box of the culture apparatus is divided into a plural number of spaces by a separator and this separator is equipped with a practicable separator door for transferring the culture container.

Further, the invention of the present application provides, in a seventh aspect, the automatic culture apparatus, wherein a unit for washing cells or a tissue is provided and transferring of the culture container to this washing unit and movement thereof are controlled by the instruction controlling unit, in an eighth aspect, the automatic culture apparatus, wherein a unit for adding a chemical is provided and transferring of the culture container to this chemical adding unit and movement thereof are controlled by the instruction controlling unit, and in a ninth aspect, the automatic culture apparatus, wherein a culture substance in the culture container is transferred into and out of the culture container while maintaining a blocking property against the outside space of the culture container.

Furthermore, the invention of the present application provides, in a tenth aspect, the automatic culture apparatus, wherein a unit for peeling or recovering a culture substance from the culture container is provided in the box of the culture apparatus and transferring of the culture container to this apparatus is made possible by the transferring unit.

The invention of the present application provides, in an eleventh aspect, the automatic culture apparatus, wherein the unit for peeling or recovering is a vibration unit or rotation unit, in a twelfth aspect, the automatic culture apparatus, wherein a press unit for changing the culture environmental conditions is provided in the box of the culture apparatus and transferring of the culture container to this apparatus is made possible by the transferring unit, and in a thirteenth aspect, the automatic culture apparatus, wherein the press unit is operated by removal and attachment of a magnet or by mechanical pressing.

The invention of the present application provides, in a fourteenth aspect, the automatic culture apparatus, wherein the container filled with a liquid culture medium, washing liquid or chemical is not re-used, in a fifteenth aspect, the automatic culture apparatus, wherein supplying of a liquid culture medium, washing liquid or chemical to the culture container is conductive via a sterilized syringe, and in a sixteenth aspect, the automatic culture apparatus, wherein supplying of a liquid culture medium, washing liquid or chemical to the culture container is conductive via a sterilized tube connected to the container for a liquid culture medium, washing liquid or chemical.

Still further, the invention of the present application also provides, in a seventeenth aspect, an automatic culture apparatus equipped with a noninvasive measurement apparatus in which the amount and/or quality of cells or a tissue with a biological origin is analyzed and measured in static adhesion culture using a culture container for culturing cells or a tissue with a biological origin, in an eighteenth aspect, the automatic culture apparatus equipped with a noninvasive measurement apparatus, wherein the culture container is equipped with electrodes for measuring electric capacity, and a culture substance is placed between two or more electrodes for measuring electric capacity and the electric capacity of the culture substance is measured, in a nineteenth aspect, the automatic culture apparatus equipped with a noninvasive measurement apparatus, wherein a displacement meter provided with an XY scanning unit is placed at an upper position of the culture container, and cells or a tissue with a biological origin is analyzed and measured based on measurement of the thickness of a cell by the displacement meter, and in a twentieth aspect, the automatic culture apparatus equipped with a noninvasive measurement apparatus, wherein a fluorometry unit provided with an XY scanning unit is placed at an upper position of the culture container, and cells or a tissue with a biological origin is analyzed and measured based on fluorometry.

### Brief Description of Drawings

Fig. 1 is a schematic view exemplifying an embodiment of a culture apparatus in which a culture operation and culture environments are automatically controlled.
Fig. 2 is a schematic view exemplifying an embodiment of a culture apparatus in which the culture apparatus in Fig. 1 is divided into a plural number of spaces by a separator.
Fig. 3 is a schematic view exemplifying an embodiment of a culture apparatus in which the culture apparatus in Fig. 1 is endowed with air control, washed, and maintained at positive pressure.
Fig. 4 is a schematic view exemplifying an embodiment of a culture apparatus of the present invention in Fig. 1, obtained by providing a recovered culture substance storing bath, a recovered culture substance transferring unit, and vibration and rotation unit.
Fig. 5 is a schematic magnification view exemplifying the vibration and rotation unit.
Fig. 6 is a schematic view exemplifying an embodiment of a culture apparatus equipped with a pressing unit in the culture apparatus in Fig. 1.
Fig. 7 is a schematic magnification view of a magnetic mode pressing unit, corresponding to the pressing unit in Fig. 6.
Fig. 8 is a schematic magnification view of an isolation mode pressing unit, corresponding to the pressing unit in Fig. 6.
Fig. 9 is a schematic view exemplifying a sterilized syringe provided in an automatic culture apparatus.
Fig. 10 is a schematic view exemplifying a sterilized tube provided in an automatic culture apparatus.
Fig. 11 is a schematic view exemplifying an embodiment of an automatic measurement apparatus by measurement of electric capacitance and a magnification view of its main portion.
Fig. 12 is a schematic view exemplifying an embodiment of an automatic measurement apparatus by measurement of by a displacement meter.
Fig. 13 is a schematic view exemplifying an embodiment of an automatic measurement apparatus by fluorometry.

Reference numerals in the drawings have the following meanings.
- 1:: culture apparatus
- 2:: culture container
- 3:: gas incubator
- 4:: gas container
- 5:: gas supplying unit
- 6:: culture container transferring unit
- 7:: fresh medium storing bath
- 8:: fresh medium supplying unit
- 9:: used medium storing bath
- 10:: used medium transferring unit
- 101:: recovered culture substance storing bath
- 102:: recovered culture substance transferring unit
- 11:: microscope observation stage
- 12:: microscope CCD camera
- 13:: observed image data transferring unit
- 14:: measured data transferring unit
- 15:: control signal transmitting unit
- 16:: control computer
- 17:: separator
- 18:: separator door
- 19:: incubate division region-a
- 20:: operation and measurement division region
- 21:: incubate division region-b
- 22:: sterilized gas generation unit
- 23:: sterilized gas introduction tube
- 24:: sterilized gas introduction valve for incubate division region-b
- 25:: sterilized gas introduction valve for operation and measurement division region
- 26:: sterilized gas introduction valve for incubate division region-a
- 27:: sterilized gas discharge valve for incubate division region-b
- 28:: sterilized gas discharge valve for operation and measurement division region
- 29:: sterilized gas discharge valve for incubate division region-a
- 30:: sterilized gas discharge tube
- 31:: sterilized gas discharge unit
- 32:: sterilization filter
- 33:: return wind tube
- 34:: discharge wind tube
- 35:: outside air incorporating tube
- 36:: return wind valve
- 37:: discharge wind valve
- 38:: outside air incorporating valve
- 39:: air circulation pump
- 40:: pressure gage in culture apparatus
- 41:: pressure signal transmitting unit
- 42:: pressure controller
- 43:: pump operation control signal
- 44:: vibration and rotation unit
- 45:: vibration generator
- 46:: rotation disc
- 47:: pressing unit of culture container
- 48:: pressing unit
- 49:: liquid culture medium
- 50:: culture substance
- 51:: weight
- 52:: electromagnet
- 53:: current circuit
- 54:: switch
- 55:: flexible culture container upper lid
- 56:: pressing substance
- 57:: action axis
- 58:: up and down driving unit
- 59:: sterile wrapping
- 60:: piston part
- 61:: cylinder part
- 62:: sterilized liquid culture medium
- 63:: cap
- 64:: liquid culture medium container
- 65:: tube
- 66:: pump
- 67:: culture container
- 68:: cell
- 69:: electrode
- 70:: capacitance meter
- 71:: XY scanning unit
- 72:: displacement meter
- 73:: laser light
- 74:: fluorometry unit
- 75:: excitation light
- 76:: fluorescence

### Best Mode for Carrying Out the Invention

The invention of the present application is characterized by a culture apparatus in which a series of culture operations for cell or tissue culture and various culture environment control operations are automated in a closed and aseptic culture apparatus, by the constitution as described above, further, characterized by division of a culture apparatus into a plural number of spaces.

The invention of the present application is also characterized by a measurement apparatus in which the property and conditions of a culture substance of cells and a tissue can be noninvasively measured and these series of measurement operations are automated.

Embodiments of the invention of the present application will be illustrated in detail below.

First, "aseptic" in the invention of the present application means a clean degree of class 1000 or less, desirably 100 or less.

Cells or tissues to be treated in an automatic culture apparatus of the invention of the present application are all derived from an organism. Here, "the organism" includes plants, insects and animals, and the animals include birds, reptiles, Amphibia, Pisces, mammals and the like. Further, examples of the mammals include human, monkey, pig, cow, sheep, mouse, horse and the like.

"Cell" to be cultured may be a culture cell with any origin, and examples thereof include plant cells, insect cells and animal cells, and may be a fused cell obtained by mutual fusing of cells with different origins or fusing of a cell with a non-cell material such as a collagen membrane, cocoon filament, micro tip, nylon mesh and the like. Of course, it may be a primary cell or established cell. Particularly, an animal cell is a preferable embodiment. Further, examples of the primary cell among animal cells include rat primary liver cells, mouse primary medulla cells, swine primary liver cells, human primary umbilical cord blood cells, human primary medulla hematopoietic cells, human primary nerve cells, and the like. Examples of the established cells include CHO cells derived from Chinese hamster ovary cells, Hela cells derived from human uterus cancer, Huh7 cells derived from human liver cancer, and the like. Cells obtained by performing gene manipulation such as plasmid introduction, virus infection and the like on these cells can also be used in the invention of the present application. "Primary cell" means, in general, a cell showing proliferation and division for only a limited time such as about 50 after being collected from an organism and "established cell" means a cell showing proliferation and separation for 50 times or more even after being collected from an organism.

On the other hand, "tissue" includes liver, heart, kidney, skin, bone, cartilage, medulla and the like, and tissues derived from these exemplified tissues.

In culturing cells, a chemical called differentiation induction factor is used in some cases as a factor to promote differentiation, for obtaining cells or tissues of any kinds, and selection of an optimum differentiation induction factor among these various differentiation induction factors depends on the kind of a cell before differentiation and the kind of a cell obtained after differentiation. The differentiation induction factors can be used singly or in combination. Examples of these differentiation induction factors include erythropoietin inducing differentiation into a red blood cell, bone morphogenic protein (BMP) promoting induction of differentiation into an osteoblast, hepatocyte growth factor (HGF) inducing differentiation into a hepatic parenchymal cell, tumor growth factor-β (TGF-β) promoting differentiation into a cartilage cell, and the like.

Origin of cells or a tissue used for transplantation, and the transplantation subject, in the invention of the present application include autogenous transplantation when both of them are the same individual, allotransplantation when both of them are the same organism species but individuals are different, or heterotransplantation when both of them are different organism species, and the like.

"Culture container" used in the invention of the present application may be made of any material, for example, plastic, glass and the like. Examples of plastic materials include natural fibers such as cellulose and the like, synthetic compounds such as polystyrene, polysulfone, polycarbonate and the like, and mixtures combining them, and the like. Organism absorbing or organism decomposition polymers such as polylactic acid, polyglucuronate and the like may also be used. Further, plastic materials may be coated with a natural extracellular matrix such as collagen, gelatin, fibronectin and the like or an artificial compound such as ethylene vinyl alcohol copolymer and the like for hydrophilization, and used as a culture container. Also, those modified with diethylamine, diethylaminoethyl and the like and those subjected to plasma discharge treatment and the like to introduce a charged group on the surface can be used.

Such a culture container may be that which is designed mainly intending use in general institutes and laboratories and is commercially available, and its content volume is, in general, 100 µL to 500 mL, and a culture container for large scale culturing can also be used. Further, a culture container containing a carrier for adhering and fixing cells or a tissue can also be used. Examples of the carrier in this case include non-woven fabric, woven fabric, gel, foamed body, cocoon filament, wire, freeze dried porous body and the like. Furthermore, there can also be used culture containers containing inside a film such as an ultrafiltration film, precise filtration film, reverse osmosis film and the like intending inhibition and promotion of movement and selection of substances or adhesion of cells and the like in a culture container.

As the shape of a culture container, dish form composed of a pan portion and a lid portion, flask form having one or more apertures for entering and discharging liquid and the like, are exemplified. Dish form culture containers include multi-well form containers obtained by dividing a space in a dish into a plural number of spaces, and flask form culture containers include those made of a porous film having an inner surface all of which or part of which has gas permeability, and these can also be used naturally.

Regarding "liquid culture medium" and "culture operation" in the invention of the present application, basically known composition components and methods can be used. Of course, these are not all examples. "Seeding operation" can also be automated by the invention of the present application. For example, it is supposed that any containers and cartridges and the like containing a culture substance are placed in a seeding unit which is a unit for seeding a culture substance into a culture container prepared in a culture apparatus, and the culture substance is poured or seeded from the seeding unit into the culture container, and the like. In "liquid culture medium", serum may or may not be contained, and if necessary, various proliferation factors and differentiation induction factors may be added and used. Examples of the proliferation factor and differentiation induction factor include an epidermal growth factor, platelet-derived growth factor, transferrin, insulin, serum albumin and the like, and extracellular matrices such as collagen, fibronectin, laminin and the like can also be exemplified. These also include a case of extraction from organs, tissues, cells and the like of organisms such as pituitary gland, luteal body, retina, kidney, thymus gland, placenta and the like, and a case of production by gene engineering such as a gene manipulation technology and the like. Further, modified bodies of these factors acting as a growth factor or differentiation induction factor are also include, and for example, those obtained by adding other amino acid to an amino acid sequence of the above-mentioned factor group, those obtained by substitution with other amino acids, those obtained by partial defective of amino acids, and the like are exemplified. Furthermore, among the above-mentioned growth factors, those of human type and other animal types may also be permissible when the type varies depending on origin.

"Addition of chemical" as "culture operation" is an operation of adding a specific culture factor to cells or a tissue during culture, and usually, means that a solution of the culture factor is poured into a liquid culture medium in a culture container. The medium factor includes serum, various growth factors and differentiation induction factors, saccharide such as glucose, saccharose and the like, antibiotics such as ampicillin, G418, tetracycline and the like, vector viruses and the like.

In the culture apparatus of the invention of the present application, a series of culture processes are automated as described above, and control of culture environments is also automated. Here, "control of culture environments" means an operation of factors affecting the metabolism, shape, function and the like of cells in culture. An operation of introducing a sterile gas for preventing contamination, and analysis operations such as observation of the shape of cells or a tissue during culture, count of cell number, activity measurement and the like are also included. Factors affecting cells or a tissue during culture include specifically the temperature of a liquid culture medium, concentration of a gas, static pressure in a liquid culture medium, speed of supplying nutrition to a liquid culture medium, concentration of wastes in a liquid culture medium, pH of a liquid culture medium, and the like. By automation of culture processes and control of culture environments, contaminations such as cross pollution between different cells and the like can be prevented, growth attitude of cells or a tissue is managed, working efficiency is improved, and the like. In the invention of the present application, "gas" used for proliferation may be any gas providing it is a gas, and preferably, a carbon dioxide gas is used.

The above-mentioned "cross pollution" means that in a plural number of divided spaces in a box of an automatic culture apparatus, a certain divided space is polluted by mixing of certain cells during culture, viruses and the like infected on the cells, and miscellaneous germs, chemical substances or, substances with other origin than that of a culture substance during culture, and the like, and an influence of this pollution is transmitted to other divided spaces, to cause pollution.

Further, the automatic culture apparatus of the invention of the present application also includes a box of a culture apparatus divided into a plural number of spaces. Here, "division" means a condition under which a space in a box of the culture apparatus can be divided into a plural number of closed spaces. Between adjacent divided spaces, for example, a separator is placed to block and close the spaces, and if necessary, the spaces can be opened and closed. The proportion of apertures occupying the surface area of the separator is supposed to be preferably 10%, further preferably less than 1%. By this, multi-lot cells or tissues can be cultured concurrently, further, the growth attitude of cells or a tissue of each lot can also be managed, furthermore, maintenances such as sterilization, washing and the like for each division are made easy, and by blocking from other divided spaces by a separator, culture and the above-mentioned maintenances can also be conducted simultaneously. By such a constitution, contaminations such as cross pollution and the like as described above can also be prevented.

The above-mentioned separator is not particularly limited in its raw material and the like providing it is made of a material capable of dividing a box of a culture apparatus into a plural number of spaces, such as metals such as stainless steel and the like, plastics or glasses and the like. This "separator" preferably has "separator door" capable of being opened and closed for smoothly conducting movement of a culture container. Further, the mechanism of opening and closing movement of this "separator door" can also be automatic by controlling the opening and closing movement under an instruction in the form of an electrical signal.

As the movement mechanism of this automatic opening and closing, use of a sensor can also be considered. For example, it is supposed that sensors detecting the weight, movement and the like of a culture container are placed near a separator door, and an instruction control unit is so set that when a culture container moves to near a separator door, the above-mentioned sensors react and the separator door automatically opens and closes.

For the above-mentioned "sterile operation", for example, a sterile gas, dry and heat sterilization, γ-ray sterilization, light sterilization and the like are mentioned, and an sterilization operation by introducing a sterile gas is preferable in view of secure sterilization of the whole space in a box of a culture apparatus, and shortening of sterilization time, and the like. Of course, this sterile gas introducing operation can also be automated by control of an instruction control unit, and the sterile gas can also be introduced arbitrarily into all or part of a plural number of divided spaces in a box of a culture apparatus. As this "sterile gas", for example, an ozone gas, ethylene oxide (EOG), vapor hydrogen peroxide (VHP), and the like are mentioned, and in view of increase in efficiency of a cell culture cycle, an ozone gas giving shorter sterilization time is preferable. The sterilization time is not particularly restricted, however, from the standpoint of desirability of increase in efficiency of a sterilization and culture cycle in the whole box of a culture apparatus, it is preferably in the range from 30 minutes to 10 hours. Particularly when the sterile gas is an ozone gas, the sterilization time is preferably in the range from 30 minutes to 1 hour.

Furthermore, the culture apparatus of the invention of the present application enables culturing of a culture substance while pressing continuously, intermittently or periodically from limited direction. By this, automatic control of pressing force which is one of culture environmental factors is made possible. As a power source of pressing, for example, gravity and magnetic force of a magnet can be utilized. Magnet may be any material providing it has magnetic force, and may be solid or liquid, and solid is preferable. Size of magnetic force may be constant, or may be arbitrarily changed like an electromagnetic.

The automatic culture apparatus of the invention of the present application is characterized in that a container filled with a liquid culture medium, washing liquid or chemical is not re-used, and characterized in that a culture substance in a culture container is transferred into and out of a culture container while maintaining a blocking property against the outside space of a culture container. Here, "not re-used" means that a liquid culture medium, washing liquid or chemical is collected from each container, and injected into one or more culture containers in one time, thereafter, each container filled with these liquid culture medium and the like is not re-used in one culture work. Each container filled with the above-mentioned liquid culture medium and the like can be used in the subsequently culture work by, after completion of one culture work, being washed and sterilized sufficiently (autoclave sterilization, UV irradiation sterilization, γ-ray sterilization, and the like), however, for surely preventing mixing and supplying into other culture container, it is more preferable that the above-mentioned each container is disposable. By this, after a liquid culture medium and the like are supplied into one or more culture containers, supplying (mixing) into a culture container culturing other cell species can be prevented. "One culture work" means a series of culture operations input into and instructed by a control computer provided in the invention of the present application, and this one operation time is not restricted since it varies depending on the object of culture, environments and the like, and it is preferably 30 minutes or less. The number of culture containers is preferably 30 or less, further preferably 5 or less. "Blocking property" means no contact with outer air (air, carbon dioxide gas and the like) outside of a culture container, or culturing instruments such as a pipette, culture container and the like. By this, a sealing property in a culture container can be further enhanced, consequently, a possibility of mixing of bacteria and the like in collecting and pouring a culture substance and a liquid culture medium and the like can be further reduced. It is also characteristic that supplying of a liquid culture medium, washing liquid or chemical to a culture container is conducted via a sterilized syringe or a sterilized tube connected to a culture container.

"Sterilized syringe" is a syringe (injection tube) sterilized by various sterilization methods, and this syringe is constituted of a container part and a piston part. The presence of an injection needle is not critical, and its raw material may be plastic (polyethylene and the like) or glass. "Sterilized tube" is a tube (tubular) sterilized by various sterilization methods like the sterilized syringe. The internal diameter thereof is preferably 0.1 mm or more and 20 mm or less. As the above-mentioned sterilization means, for example, irradiation with γ-ray, sterile gas, autoclave and the like are mentioned. These sterilized syringe and sterilized tube are preferably disposable, to prevent mutual mixing between culture containers.

The invention of the present application enables analysis and measurement of the amount and/or quality of cells or tissue, based on electric capacitance measurement, displacement meter measurement or emission measurement, using various units equipped with an XY scanning unit at an upper position of a culture container. Additionally, this analysis measurement enables observation and analysis measurement noninvasively on cells or a tissue.

For the above-mentioned "electric capacitance measurement", there are, for example, a method in which a difference in phase of electric current period caused by electric capacitance in periodically applying voltage on a culture container (culture container contains cells or a tissue during culture) placed between two or more electrodes is detected, and electric capacitance is calculated, and other methods. A plasma membrane present in a cell has a lipid double membrane and is an insulator, consequently, has dielectric constant. When voltage is applied on a cell wrapped with a lipid double membrane in a liquid culture medium containing salts, the magnitude of electric capacitance is in proportion to the surface area of a plasma membrane and in inverse proportion to the thickness of a plasma membrane, therefore, by measuring electric capacitance, the size and thickness of a cell can be measured.

For "displacement meter measurement", units based on various theories are used, and for example, a laser displacement meter irradiating a measuring article (cell or tissue and the like during culture) with laser ray and measuring reflected light, wavelength confocal method utilizing confocal of visible light, and the like can be adopted, however, these are not all examples.

In "fluorometry", for example, properties, shapes and the like of cells or a tissue and the like are measured by detecting a substance emitting fluorescence (fluorescent substance) such as a fluorescent coloring matter compound, fluorescent protein, fluorescent semiconductor (quantum dot) and the like. As the fluorescent substance, preferable are fluorescent proteins, and particularly, intrinsic fluorescent proteins are preferable. For example, NADH essentially present in a cell, and the like are mentioned. As the extrinsic fluorescent protein, for example, a green fluorescent protein, red fluorescent protein, yellow fluorescent protein, blue fluorescent protein and the like are mentioned. The excitation light wavelength and excitation light intensity of a fluorescence measuring apparatus are not particularly restricted, however, the excitation light wavelength is preferably in the range from 300 nm to 600 nm, for example, and the excitation light intensity is, in view of damage on a cell, preferably 100 mW or less. As the fluorescence measuring method, a photomulti-plier mode, CCD mode and the like are exemplified, and these can be appropriately selected.

These measuring methods are automated by computer control, and can observe and analyze cells or a tissue noninvasively and three-dimensionally. "Noninvasive" means that load on cells or a tissue is extremely small, without requiring contact with cells or a tissue as a culture substance and without requiring a destructive operation such as homogenization and the like, and "three-dimensional" means not only two-dimensional information but also three-dimensional information on formation, shape and the like of cells or a tissue, namely, the thickness, shape and the like of cells. "Amount and/or quality" of cells or a tissue means the degree of proliferation or the degree of growth of cells or a tissue being cultured, alternatively, the shape thereof and various secreted substances and the like.

Next, embodiments of the invention of the present application will be illustrated further in detail referring to appended drawings, however, the invention of the present application is not limited to these examples. In the appended drawings, a gas container 4 is placed in a culture apparatus 1, for example, however, it may be placed outside of a culture apparatus 1. Though not shown, it is also possible to place a centrifugal separator and the like in the culture apparatus 1 as an apparatus for separating cells and a liquid culture medium from a cell-containing liquid culture medium.

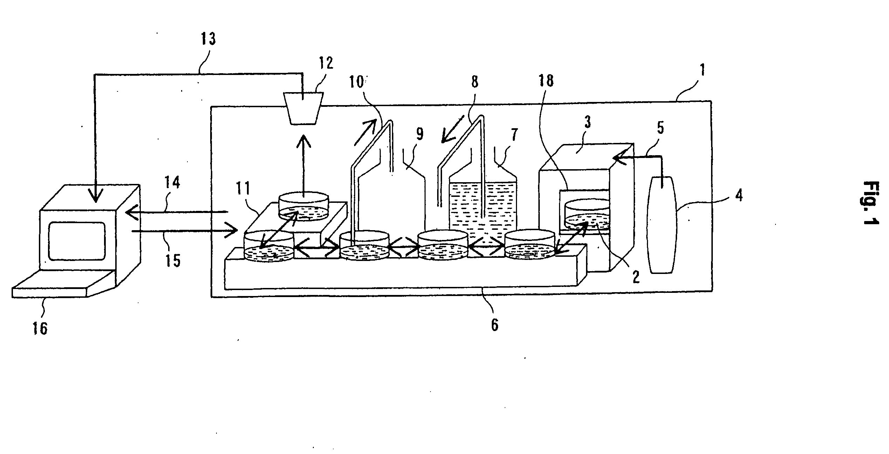

Fig. 1 exemplifies an embodiment in which a culture process and culture environment control are automated. In this example of Fig. 1, in the culture apparatus 1 in the form of box having a closed space, the gas concentration can be kept constant by a gas container 4 and a gas supplying unit 5, and a gas incubator 3 in the form of box in which a culture container 2 having a culture substance can be allowed to stand still is present, and cells or a tissue is cultured in the culture container 2 allowed to stand still in this. Further, this gas incubator 3 is provided with a practicable separator door 18 for smoothly transferring the culture container 2 into or out of the gas incubator 3. The culture container 2 during culture moves in the culture apparatus 1 forwardly or backwardly arbitrarily by a culture container transferring unit 6. In this transferring process, by controlling the position for liquid culture medium suction by a fresh medium supplying unit 8 in a fresh medium storing bath 7, a certain amount of liquid is sucked, and a liquid culture medium is poured into the culture container 2, and the supernatant of a liquid culture medium in the culture container 2 is, while the lower end of the used medium transferring unit 10 is inserted in the culture container 2, discharged by being sucked into the used medium storing bath 9 having an inner pressure kept at lower level, thus a liquid culture medium can be supplied and exchanged continuously or intermittently. Though not shown, by placing also a storing bath containing washing liquid such as buffer liquid and the like or a storing bath containing chemical in the culture apparatus 1, washing liquid and chemical can be added to a liquid culture medium. Of course, repetition of these processes or skipping thereof can be optionally controlled by the culture container transferring unit 6 and control computer 16. Further, the culture container 2 can be transferred also to a microscope stage 11, and for example, it is observed by using a microscope CCD camera 12, and the observed image data is transmitted to the control computer 16 via an observed image data transferring unit 13. Data of culture environments such as temperature, humidity, gas concentration and the like is transmitted from the culture apparatus 1 via a measured data transferring unit 14 to the control computer 16 in the same manner, and calculation is performed based on this data, and data for movement of the culture container 2, opening and closing of the separator door 18, and temperature control, and control signals for control of the gas concentration, are transmitted to the culture apparatus 1 through a control signal transmitting unit 15. Thus, since almost all culture operations are conducted in the culture apparatus 1, there is an extremely low possibility of pollution with a chemical, dust, miscellaneous germs and the like from outside of the culture apparatus.

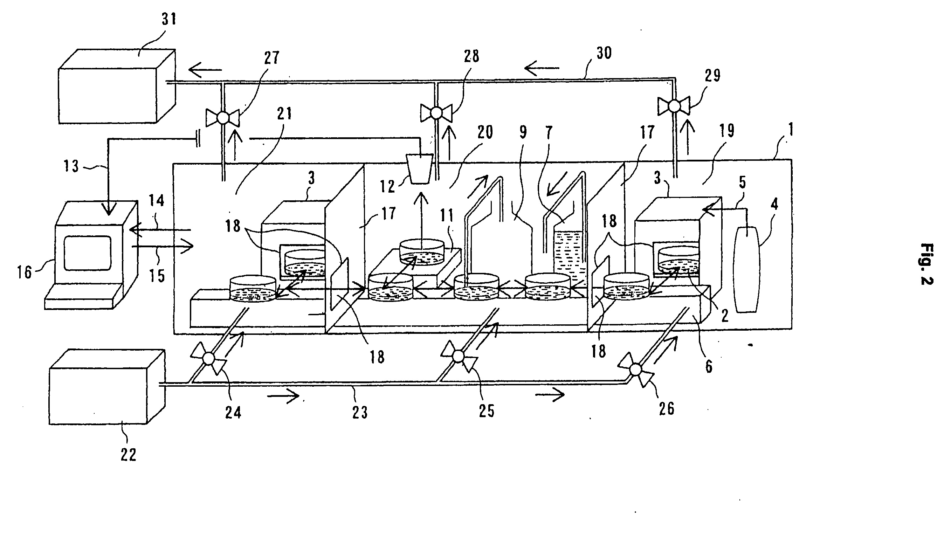

Fig. 2 exemplifies an embodiment in which the culture apparatus 1 exemplified in Fig. 1 is divided into a plural number of spaces by a separator 17. A space in the culture apparatus 1 is compartmented into three spaces, that is, an incubate division region-a 19, an operation and measurement division region 20 and an incubate division region-b 21. The culture container 2 can move forwardly or backwardly optionally to each space through the practicable separator door 18 on the separator 17, namely, it can go to and fro between three spaces. By closing the separator door 18, three spaces are mutually closed, to give independent spaces. A sterile gas is injected from a sterile gas generation unit 22 into optional spaces via a sterile gas introduction tube 23 and sterile gas introduction valves 24, 25 and 26, thus, sterilization can be performed. Further, when injecting a sterile gas into the incubate division region-a 19 exemplified in this drawing, the separator door 18 of the gas incubator 3 is opened and closed optionally, thus, a sterile gas can be injected also in the gas incubator 3. The sterile gas injected into a space during sterilization does not exert any influence since this gas is not mixed at all into other spaces. After completion of sterilization, a sterile gas is discharged into a sterile gas discharge unit 31 via sterile gas discharge valves 27, 28 and 29 and a sterile gas discharge tube 30 in spaces under sterilization, and the sterile gas does not remain in the spaces. By this, even in a culture period of long time, a space in a culture apparatus can be sterilized at any moment and extremely clean environments can be maintained. This opening and closing of the separator door 18 and sterile gas operation can also be arbitrarily controlled by the control computer 16, additionally, opening and closing can be conducted automatically. By regulating the number of the separator 17, the number of spaces to be divided can also be regulated, and resultantly, a lot of culture operation processes can be conducted simultaneously, and culture cells of many lots can be cultured.

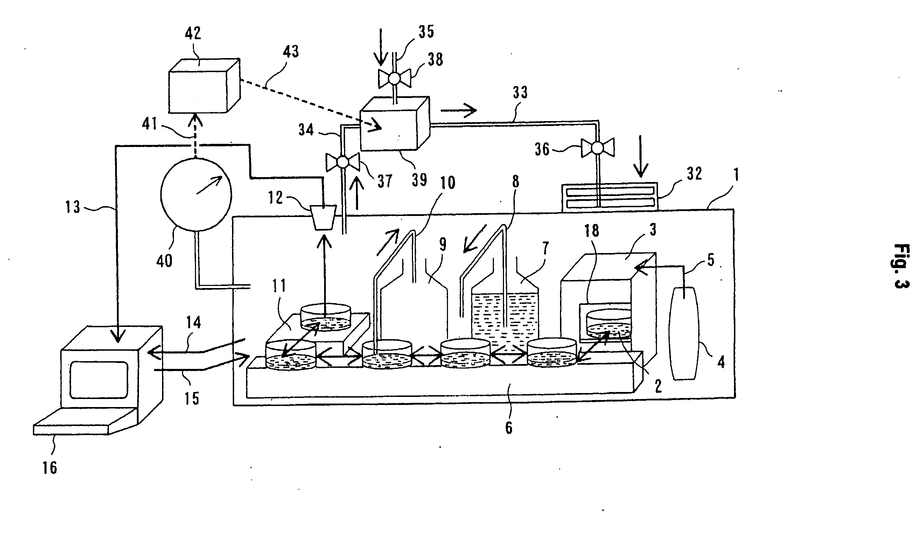

Fig. 3 exemplifies an embodiment of the culture apparatus 1 equipped with a function of pressure control. In this example, a gas in the culture apparatus 1 is transferred to an air circulation pump 39 via a discharge wind tube 34 and a discharge valve 37. Here, a gas outside of the culture apparatus 1 is also incorporated via an outer gas incorporation tube 35 and an outer gas incorporation valve 38, and a gas combining both the gases is returned into the culture apparatus 1 through a return wind tube 33 and a return wind valve 36, further, through a sterilization filter 32. By this circulation of a gas, fresh air is maintained in the culture apparatus 1. The static pressure in the culture apparatus 1 is measured by a culture container internal pressure meter 40, and its data is transmitted to a pressure controller 42 via a pressure signal transmitting unit 41. For maintaining the pressure in the culture apparatus 1 at a positive level higher than that outside, the pressure controller 42 computes and calculates set values of discharge wind speed, outer gas incorporation speed, return wind speed and the like, and transmits the results as a pump operation control signal 43 to an air circulation pump 39. As a result, air in the culture apparatus 1 is constantly kept clean, and endowed with a positive pressure higher than that outside the culture apparatus. Thus, mixing of pollution substances from outside of the culture apparatus can also be suppressed extremely effectively. Fig. 3 gives an example in which positive pressure is controlled in the pressure controller 42, however, when the control computers 16 are further connected, control of the pressure controller 42 by the control computer 16 is made possible, and through this control, control of positive pressure is also made possible.

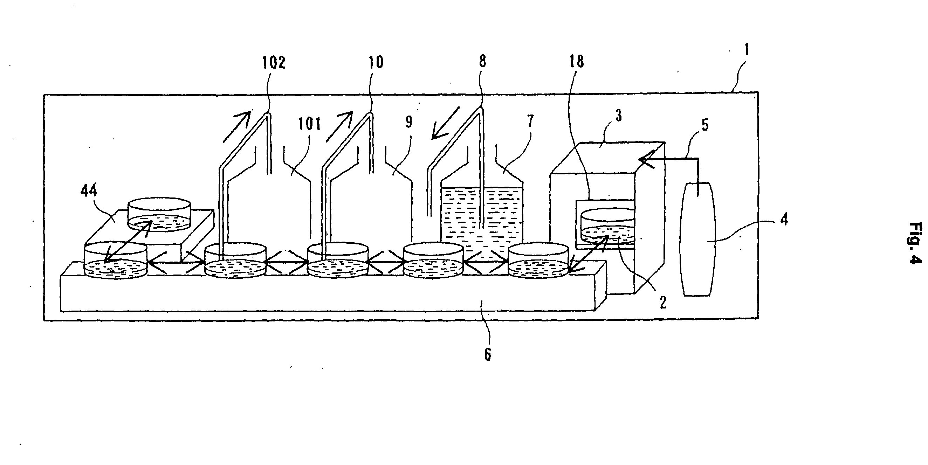

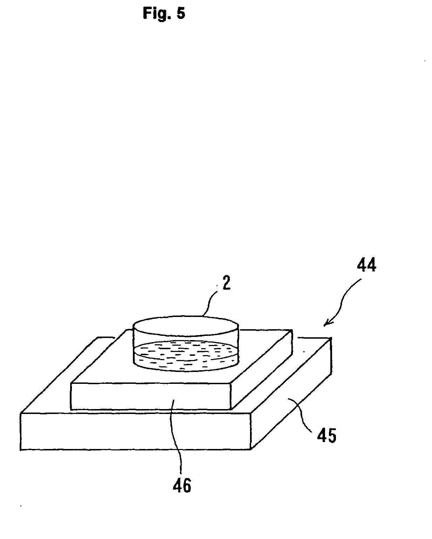

Fig. 4 exemplifies an embodiment in which the culture apparatus 1 comprises a recovered culture substance storing bath 101, recovered culture substance transferring unit 102 and vibration and rotation unit 44. In Fig. 4, illustrations of the microscope CCD camera 12, observed image data transmitting unit 13 and control computer 16 are omitted, however, the functional action as the microscope CCD camera 12 and observed image data transmitting unit 13, and mechanisms of controlling environments by control computer 16 in the culture apparatus 1, are working. Cells in the culture container 2 tend to be detached from the cell adhesion surface in the culture container, by a series of culture operations such as detaching and peeling of cells and the like by medium substitution, washing and enzymatic treatment. However, not all of cells are detached, and cells are mutually aggregated in many cases. The culture container 2 under this condition is transferred to the vibration and rotation unit 44 by the culture container transferring unit 6, and vibration and rotation are imparted to the container optionally, thus, almost all cells are detached and peeled, aggregated cells are also dissociated, resultantly, a cell suspension containing uniformly dispersed cells can be obtained. The culture container 2 containing this cell suspension is transferred to the recovered culture substance storing bath 101 by the culture container transferring unit 6, and recovered into the recovered culture substance storing bath 101 via the recovered culture substance transferring unit 102. As illustrated in Fig. 5 as a magnification schematic view, with the vibration and rotation apparatus 44, vibration is transferred to the culture container 2 by a vibration generator 45, and rotation can be imparted to the culture container 2 by a rotation disc 46.

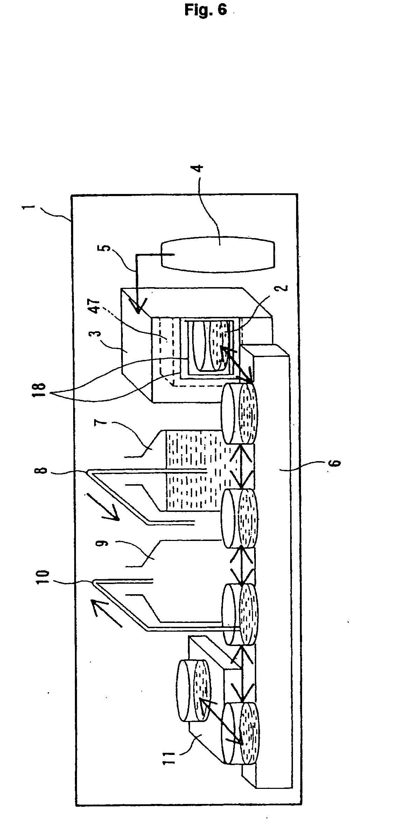

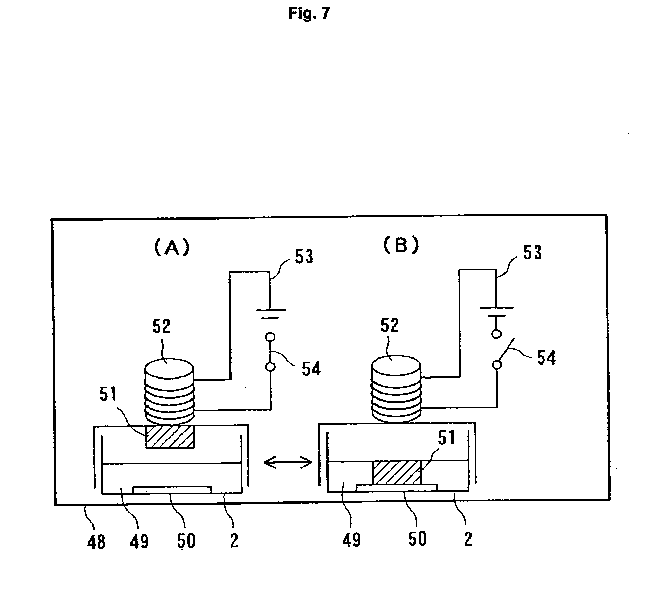

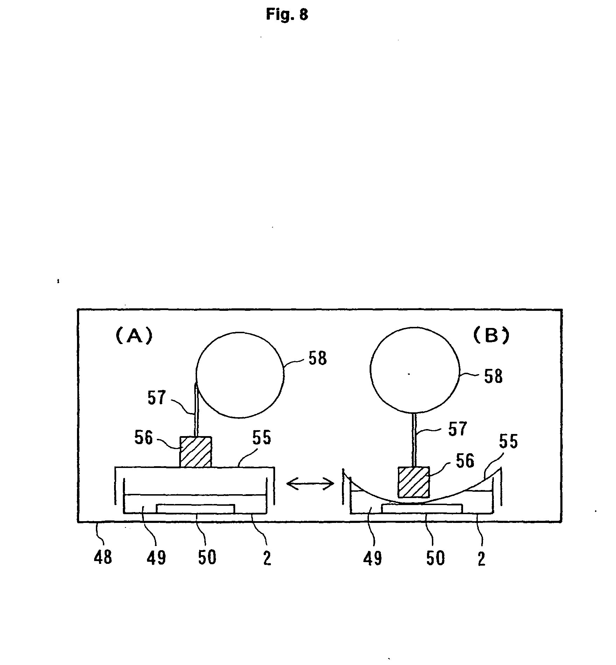

Fig. 6 shows an example of the culture apparatus 1 equipped with a culture container pressing unit 47. Though illustrations of the microscope CCD camera 12, observed data transmitting unit 13 and control computer 16 are omitted, it is of course possible to set and use them. In this example, in the gas incubator 3 in the culture apparatus 1, pressing force of any strength can be applied at any period on a culture substance in the culture container 2 during culture by the culture container pressing unit 47. By applying such pressing force, mechanisms of controlling activation, suppression, secretion and the like of various organism factors such as interleukin, cytokine, TNF-α, kinase and the like work by a stimulus of pressing force, and this stimulus signal is sequentially transmitted (cascaded) on molecule level, and resultantly, an action effect on growth of cells or a tissue is obtained, thus, culture environments for cells or a tissue can be controlled. Further, in the case of three-dimensional culture, it is also expected that a culture substance is elongated by pressing, and by this, flow of a liquid culture medium is caused forcibly between outside and inside of a culture substance, transfer of a substance is promoted. The pressing unit 47 of a culture container can be operated by various methods and mechanisms, and for example, the following Figs. 7 and 8 show examples thereof.

Fig. 7 shows a schematic structure view of a culture container pressing unit 48 in the case of magnetic mode. A weigh 51 is placed on the inside of an upper lid of the culture container 2 in the pressing unit 48, and on an upper lid of the culture container 2, an electromagnet 52 is placed, and on and off of its magnetic force can be controlled automatically by the control computer 16 by an electric current circuit 53 and a switch 54. As shown in Fig. 7 (A), when the electric current circuit is on, the weigh 51 is attracted to the electromagnet 52 placed on an upper position in the culture container 2 by the action of electromagnet, and isolated from a culture substance 50 in a liquid culture medium 49 in the culture container 2. As shown in Fig. 7 (B), when the electric source is off, the action of electromagnet disappears, and the weight 51 falls on the culture substance 50, and pressing force is applied on the culture substance 50.

Fig. 8 exemplifies a schematic structure view of the culture container pressing unit 48 in the case of isolation mode, and a flexible culture container upper lid 55 of the culture container 2 present in the pressing unit 48 is made of soft material having flexibility. On the other hand, on an upper position outside of the culture container 2, a pressing substance 56 can be moved up and down arbitrarily and automatically by the control computer 16, by the action of an up and down driving unit 58 and an action axis 57. As shown in Fig. 8 (B), when this pressing substance 56 falls, it pushes up the flexible culture container upper lid 55, and presses a culture substance 50 in a liquid culture medium 49 in the culture container 2. As shown in Fig. 8 (A), when the pressing substance 56 moves upwardly, the pressing substance 56 is lifted, and the flexible culture container upper lid 55 returns to usual upper lid shape, and the culture substance 50 is not pressed. Namely, by up and down movement of the pressing substance 56, an action of pressing the culture substance 50 is controlled.

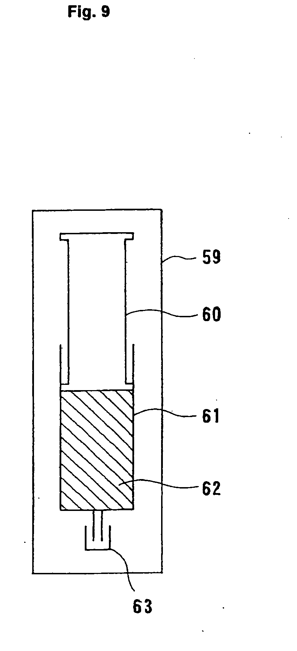

Fig. 9 is a schematic view exemplifying a sterilized syringe provided in an automatic culture apparatus. This sterilized syringe is composed of a piston portion 60 and cylinder portion 61, and a sterilized liquid culture medium 62 is placed in the cylinder and the cylinder is sealed by a cap 63, further, a wrapping 59 is provided to keep a sterile property. Instead of the fresh culture storing bath 7, a plurality of (according to demanded amount) of sterilized syringes are placed in the automatic culture apparatus 1 while maintaining the wrapped condition, and the sterilized syringes are held by a robot arm (not shown) and the like, and transferred to a desired position, and by applying suitable pressure on the piston portion 60, the sterilized liquid culture medium 62 is injected into the culture container.

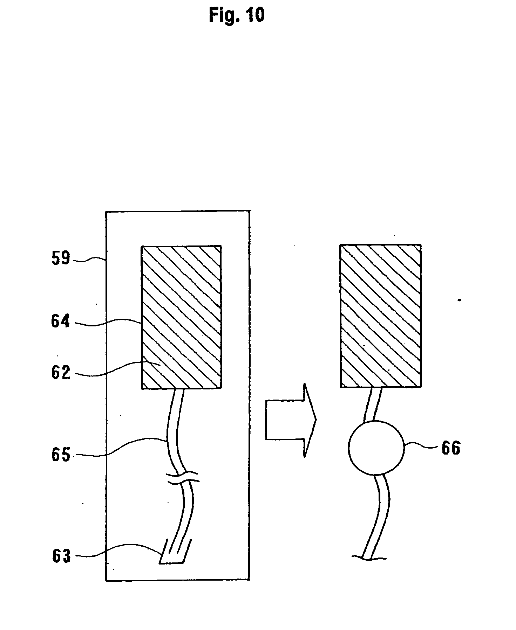

Fig. 10 is a schematic view exemplifying a sterilized tube placed in an automatic culture apparatus. This sterilized tube is composed of a liquid culture medium container 64 and a tube 65, and a liquid culture medium 62 is placed in the liquid culture medium container 64, the tip of the tube 65 is sealed by a cap 63, further, a wrapping 59 is provided to maintain a sterile property. Like the above-mentioned sterilized cylinder, instead of the fresh culture storing bath 7, a plurality of (according to demanded amount) of sterilized tubes are placed in the automatic culture apparatus 1 while maintaining the wrapped condition, and the sterilized tubes are held by a robot arm (not shown) and the like, and transferred to a desired position, and by applying suitable pressure on a pump 66 mounted later, the sterilized liquid culture medium 62 is injected into the culture container.

The above-mentioned wrapping 59 and cap 63 for the sterilized syringe and sterilized tube are removed by a robot arm and the like directly before use in the automatic culture apparatus 1. The capacity of the liquid culture medium 62 corresponds to the capacity of one to several culture containers, and after use, the liquid culture medium 62 is discarded, therefore, mutual mixing between culture containers of different cell species can be prevented.

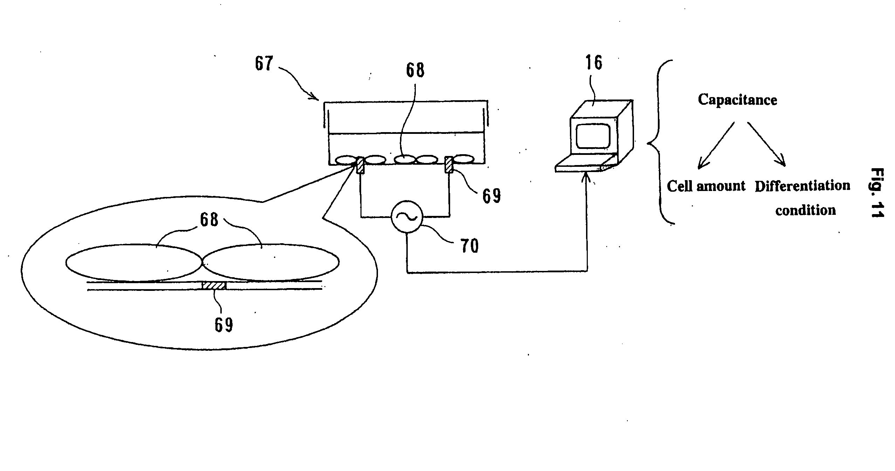

Fig. 11 provides a schematic view exemplifying an embodiment of an automatic measuring unit by measurement of electric capacitance, and an enlarged view of its main part. In a culture container 67, cells 68 are culture, and at the bottom of the culture container 67, two electrodes 69 are provided. These electrodes 69 are connected to a capacitance meter 70, and connected to the control computer 16. The capacitance value obtained by this capacitance meter 70 varies depending on the shape and condition of the cells 68 adhered to the culture container 67, and its measurement information is transmitted to the control computer 16, and analyzed and the amount and/or quality of cells such as cell amount, differentiation condition and the like are measured.

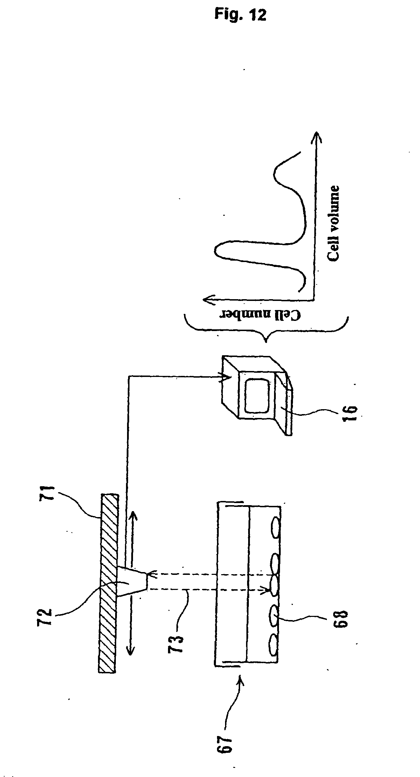

Fig. 12 is a schematic view exemplifying an embodiment of an automatic measuring unit by measurement by a displacement meter. As shown in Fig. 12, for example, those utilizing a light source can be used. On an upper position of the culture container 67, a displacement meter 72 mounted to the XY scanning unit 71 is present, and cells are irradiated with laser light 73 from the displacement meter 72, and its light is reflected to the displacement meter 72. By measuring a difference in reflection from the upper position of cells and the lower position of cells, the thickness of the cells can be measured. Of course, the displacement meter is not limited to those utilizing a light source.

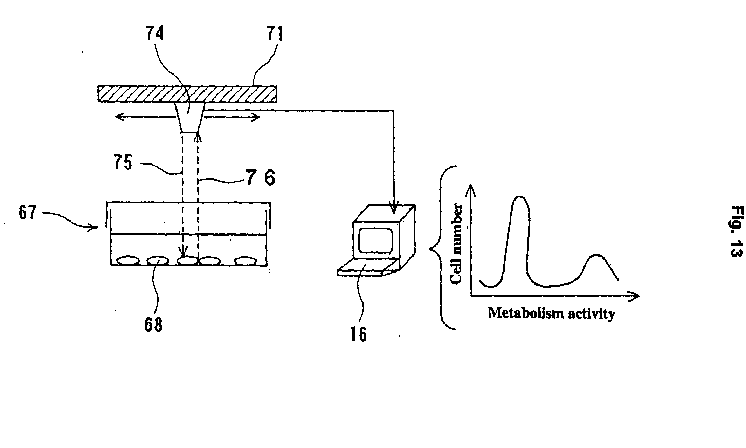

Fig. 13 is a schematic view exemplifying an embodiment of an automatic measuring unit by measurement of fluorescence. In Fig. 13, cells 68 are adhesion-cultured in the culture container 67, and on an upper position of the culture container 67, a fluorescence measuring unit 74 mounted to the XY scanning unit 71 is present, and the cells 68 are irradiated with excitation light 75 from this fluorescence measuring unit 74. Fluorescence 76 emits from the cells 68, and this fluorescence 76 is measured by the fluorescence measuring unit 74, and differentiation condition and the like of the cells 68 can be analyzed and measured.

In any of the above-mentioned measuring units, various information (form, proliferation speed, observation of secreted substance, and the like) on cells or tissues placed in the automatic culture apparatus 1 and obtained from this measuring unit, are transmitted to and analyzed by the control computer 16, and further, automatically controlled by this control computer.

### Industrial Applicability

According to the invention of the present application, a culture apparatus is provided in which almost all culture processes are automated, a special sterilization facility is not necessary, extremely clean sterile environments are prepared, further, factors of culture environments are not restricted, and automatic control is possible depending on culture progress.

Since a space in the culture apparatus of the invention of the present application is divided in a plural number of culture chambers, a culture apparatus is provided in which proliferation attitudes varying for every lots can be managed, and many lots can be concurrently cultured in small scale, further, maintenances such as washing, disinfection and the like can be conducted separately, and culturing for a long period of time is possible since mutual mixing between different cell species and tissue species can also be prevented.

Furthermore, the invention of the present application provides an automatic culture apparatus equipped with a measuring unit in which a series of operations such as observation, measurement, analysis and the like are automated noninvasively and three-dimensionally on cells or a tissue to be cultured.

## Claims

1. An automatic culture apparatus for culturing cells or a tissue with a biological origin in a culture container which is placed in a box-type culture apparatus having a closed and aseptic inner space, **characterized in that** a plural number of divided spaces, a gas incubator provided with a practicable window, a unit for supplying a liquid culture medium and a unit for discharging the same, a unit for monitoring the culture conditions, and a transferring unit for continuously or intermittently transferring the culture container toward these units are provided in the box of the culture apparatus, and at least one of the above-described units is equipped with an instruction controlling unit by which the culture apparatus is controlled under an instruction in the form of an electrical signal depending on a data signal generated by the culture condition-monitoring unit so as to prevent cross-contamination.

2. The automatic culture apparatus according to Claim 1, wherein the apparatus has a setting unit for introducing a sterile gas into all portions or partial portions in the box of the culture apparatus.

3. The automatic culture apparatus according to Claim 2, wherein the sterile gas is an ozone gas.

4. The automatic culture apparatus according to any of Claims 1 to 3, wherein the apparatus has an environmental condition setting unit for allowing all portions or partial portions in the box of the culture apparatus to have a positive pressure higher than that of the outer space.

5. The automatic culture apparatus according to any of Claims 1 to 4, wherein the box of the culture apparatus is divided into a plural number of spaces and the spaces are mutually closable.

6. The automatic culture apparatus according to Claim 5, wherein the box of the culture apparatus is divided into a plural number of spaces by a separator and this separator is equipped with a practicable separator door for transferring the culture container.

7. The automatic culture apparatus according to any of Claims 1 to 6, wherein a unit for washing cells or a tissue is provided and transferring of the culture container to this washing unit and movement thereof are controlled by the instruction controlling unit.

8. The automatic culture apparatus according to any of Claims 1 to 7, wherein a unit for adding a chemical is provided and transferring of the culture container to this chemical adding unit and movement thereof are controlled by the instruction controlling unit.

9. The automatic culture apparatus according to any of Claims 1 to 8, wherein a culture substance in the culture container is transferred into and out of the culture container while maintaining a blocking property against the outside space of the culture container.

10. The automatic culture apparatus according to any of Claims 1 to 9, wherein a unit for peeling or recovering a culture substance from the culture container is provided in the box of the culture apparatus and transferring of the culture container to this apparatus is made possible by the transferring unit.

11. The automatic culture apparatus according to Claim 10, wherein the unit for peeling or recovering is a vibration unit or a rotation unit.

12. The automatic culture apparatus according to any of Claims 1 to 11, wherein a press unit for changing the culture environmental conditions is provided in the box of the culture apparatus and transferring of the culture container to this apparatus is made possible by the transferring unit.

13. The automatic culture apparatus according to Claim 12, wherein the press unit is operated by removal and attachment of a magnet or by mechanical pressing.

14. The automatic culture apparatus according to any of Claims 1 to 13, wherein the container filled with a liquid culture medium, washing liquid or chemical is not re-used.

15. The automatic culture apparatus according to any of Claims 1 to 14, wherein supplying of a liquid culture medium, washing liquid or chemical to the culture container is conductive via a sterilized syringe.

16. The automatic culture apparatus according to any of Claims 1 to 14, wherein supplying of a liquid culture medium, washing liquid or chemical to the culture container is conductive via a sterilized tube connected to the container for a liquid culture medium, washing liquid or chemical.

17. An automatic culture apparatus equipped with a noninvasive measurement apparatus in which the amount and/or quality of cells or a tissue with a biological origin is analyzed and measured in static adhesion culture using a culture container for culturing cells or a tissue with a biological origin.

18. The automatic culture apparatus equipped with a noninvasive measurement apparatus according to Claim 17, wherein the culture container is equipped with electrodes for measuring electric capacity, and a culture substance is placed between two or more electrodes for measuring electric capacity and the electric capacity of the culture substance is measured.

19. The automatic culture apparatus equipped with a noninvasive measurement apparatus according to Claim 17, wherein a displacement meter provided with an XY scanning unit is placed at an upper position of the culture container, and cells or a tissue with a biological origin is analyzed and measured based on measurement of the thickness of a cell by the displacement meter.

20. The automatic culture apparatus equipped with a noninvasive measurement apparatus according to Claim 17, wherein a fluorometry unit provided with an XY scanning unit is placed at an upper position of the culture container, and cells or a tissue with a biological origin is analyzed and measured based on fluorometry.
